# EUROPEAN PATENT SPECIFICATION

(11) **EP 3 641 860 B1**
(45) Date of publication and mention of the grant of the patent: **07.10.2026**
(21) Application number: 18731058.6
(22) Date of filing: 11.06.2018
(51) Int. Cl.: A61M 5/32

(54) **NEEDLE SHIELD REMOVER AND A MEDICAMENT DELIVERY DEVICE COMPRISING THE NEEDLE SHIELD REMOVER**
NADELSCHUTZENTFERNER UND EINE MEDIKAMENTENAUSGABEVORRICHTUNG MIT DEM NADELSCHUTZENTFERNER
DISPOSITIF DE RETRAIT DE PROTECTION D'AIGUILLE ET DISPOSITIF D'ADMINISTRATION DE MÉDICAMENT COMPRENANT LE DISPOSITIF DE RETRAIT DE PROTECTION D'AIGUILLE

(30) Priority: 23.06.2017 US 201715631319
(43) Date of publication of application: 29.04.2020
(73) Proprietor: SHL Medical AG, 6302 Zug (CH)
(72) Inventor: STEWART, Justin, Deerfield Beach 33441 (US); ELMÉN, Gunnar, 141 31 Huddinge (SE); BJÖRK, Emil, 117 68 Stockholm (SE)
(86) International application number: PCT/EP2018/065366
(87) International publication number: WO 2018/234079

(56) References cited:
- EP-A1- 2 878 321
- WO-A1-2013/058697

## Description

### TECHNICAL FIELD

The present disclosure relates to a needle shield remover and in particular to a symmetric needle shield remover for a cap of a medicament delivery device.

### BACKGROUND

Today's medicament delivery devices may be complex and involve many different components. Assembly of such devices is time consuming and is often performed manually by trained personnel. The various components are picked from trays and assembled together by orienting the components in relation to each other, and fitting them together. Often different members of one component need to be matched to guides and/or attachments means of one or more other components, sometimes while the components are under bias of a spring. Depending on the complexity of the device, the assembly steps may be numerous. It would therefore be advantageous if the assembly process could be simplified, such as by improving the design of certain components to aid in their assembly.

The present disclosure relates to a needle shield remover. The needle shield remover is often assembled with a cap, or closure member, of a medicament delivery device and is engaged to a needle shield, which protects a needle by hermetically enclosing it. The shield may comprise a flexible rubber member in which the needle is embedded, i.e. a flexible needle shield (FNS). For some applications, the FNS is provided with a rigid outer shell, i.e. a rigid needle shield (RNS). To be able to remove the needle shield before use of the medicament delivery device, the needle shield remover is provided with gripping members that engage the needle shield. Since the needle shield remover is attached to the cap, removal of the cap will also pull away the needle shield from the needle, due to the gripping members' engagement with the needle shield.

An example of a needle shield remover is disclosed in WO2013/058697, wherein a remover body is provided with two different sets of gripping members at a distal part of the remover body. One set of gripping members is configured to be able to engage a FNS and the other set of gripping members is configured to be able to engage a RNS. Both sets of gripping members are arranged on distal part of the remover body. A circumferentially extending ledge or protrusion is provided on a proximal end of the remover body to cooperate with a circumferential groove of a lid piece of a cap, such that the remover body may be assembled with the cap. However, the assembly operation requires the operator to determine which end of the remover body that is to be connected to the lid piece of the cap. The operation is especially time consuming if the needle shield removers are picked from a bulk transport box, instead of a tray containing pre-oriented components.

Document EP 2 878 321 discloses a needle safety device comprising a needle cap arranged to cover and protect a needle. A needle shield is relatively movable with respect to a drug delivery device to cover and protect the needle and a needle cap remover. The needle cap remover has an outer part, which is fixed to the needle shield, and an inner part adapted to carry the needle cap. The outer part comprises a front end with an opening, which at its edge exhibits a projection directed, inwards adapted to catch a front projection of the inner part. The inner part comprises a back end having a grip member adapted to grip a back end of the needle cap.

### SUMMARY

In the present disclosure, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", with or without "axis", refers to a direction or an axis through the device or components thereof in the direction of the longest extension of the device or the component.

The term "lateral", with or without "axis", refers to a direction or an axis through the device or components thereof in the direction of the broadest extension of the device or the component. "Lateral" may also refer to a position to the side of a "longitudinally" elongated body.

In a similar manner, the terms "radial" or "transversal", with or without "axis", refers to a direction or an axis through the device or components thereof in a direction generally perpendicular to the longitudinal direction, e.g. "radially outward" would refer to a direction pointing away from the longitudinal axis.

Also, if nothing else is stated, in the following description wherein the mechanical structure of the device and the mechanical interconnection of its components is described, the device is in an initial non-activated or non-operated state.

In view of the foregoing, a general object of the present disclosure is to provide a needle shield remover for a cap of a medicament delivery device, which needle shield remover is easier to assemble.

According to a main aspect of the disclosure it is characterised by a needle shield remover for assembly with a cap of a medicament delivery device, wherein the needle shield remover comprises a longitudinally elongated tubular body having a plurality of structural features, wherein said plurality of structural features are symmetrically disposed on the tubular body, in relation to a plane of symmetry which is perpendicular to a longitudinal axis.

The structural features, such as gripping members and cap fastening members, are symmetrically arranged such that the assembly of the needle shield remover with a the cap can be performed without first orienting the needle shield in a particular longitudinal direction.

The plane of symmetry is located at mid-length of the tubular body.

The plurality of structural features comprises a first cap fastening member and a second cap fastening member symmetrically disposed, in relation to the plane of symmetry, at a proximal part of the tubular member and at a distal part of the tubular member, respectively.

As such, the proximal cap fastening member may be used for attachment of the needle shield remover to a cap, if it is oriented towards the cap when picked from a tray, or box, of components. Similarly, the distal cap fastening member may be used for attachment of the needle shield remover to a cap if it is oriented towards the cap when picked from a tray, or box, of components.

The plurality of structural features comprises first shield gripping members and second shield gripping members symmetrically disposed on the tubular body proximally of the plane of symmetry and distally of the plane of symmetry, respectively.

### BRIEF DESCRIPTION OF THE DRAWINGS

- Fig. 1: a cross-sectional view of a prior art cap, needle shield remover, and syringe.
- Fig. 2: a perspective view of a needle shield remover according to a first embodiment of the present disclosure.
- Fig. 3: a perspective view of a needle shield remover according to a second embodiment of the present disclosure.
- Fig. 4: an exploded perspective view of a cap and a needle shield remover according to a third embodiment of the present disclosure.
- Fig. 5: a cross-sectional view of a cap and a needle shield remover according to a third embodiment of the present disclosure.

### DETAILED DESCRIPTION

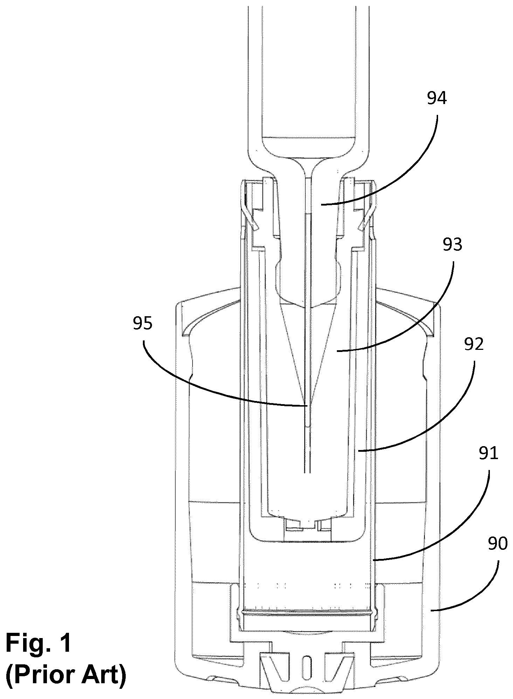

Fig. 1 shows a known needle shield remover 91 comprised in a cap 90. The cap 90 and the needle shield remover 91 are arranged on a rigid needle shield, or RNS 92, which covers a flexible needle shield, or FNS 93. A needle 95, of a syringe 94, is embedded in the FNS 93. The syringe 94 may be housed in a medicament delivery device (not shown), for instance an auto-injector. The needle shield remover 91 is attached to the cap 90 and comprises inwardly-projecting gripping members which engage the RNS 92, either by a distal end thereof or by engagement to a circumferential surface of the RNS, such that removal of the cap pulls the RNS and the FNS away from the needle 95.

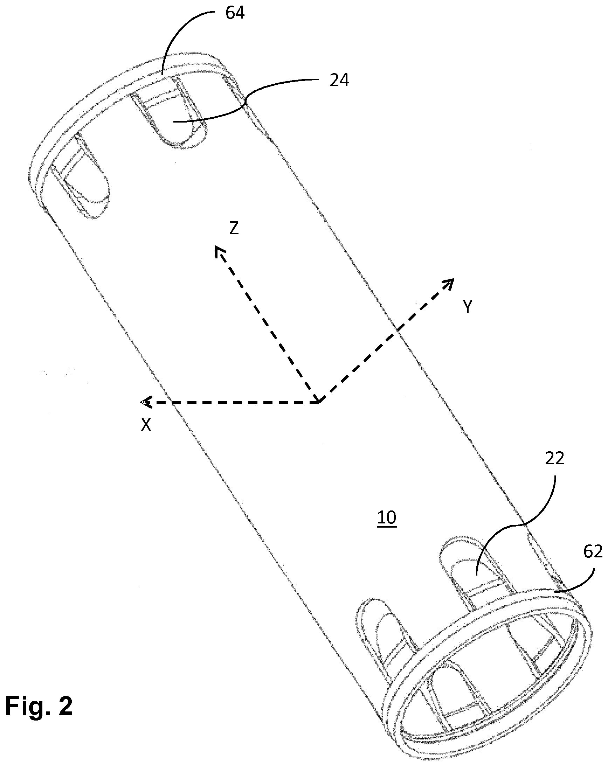

The present disclosure relates to a needle shield remover which is intended to be applied to known needle shields, for instance needle shields as shown in Fig. 1. The needle shield remover of the present disclosure is described in detail in conjunction with Figs 2-5 below.

Fig. 2 shows a needle shield remover according to a first embodiment of the present disclosure. The needle shield remover comprises a longitudinally elongated tubular body 10, elongated along an axis Z. The tubular body has a plurality of structural features. The structural features are symmetrically disposed on the tubular body, in relation to a plane of symmetry which is perpendicular to the longitudinal axis. The plane of symmetry is the X-Y plane, which is perpendicular to the Z- axis.

The plane of symmetry is preferably located at mid-length of the tubular body 10. Because the plurality of structural features is disposed symmetrically in relation to the plane located at mid-length if the tubular body 10, it is possible to longitudinally orient the tubular body in either direction when assembling the needle shield remover with a second component, such as with a cap of a medicament delivery device.

The plurality of structural features may comprise a first cap fastening member 62 and a second cap fastening member 64 symmetrically disposed, in relation to the plane of symmetry, at a proximal part of the tubular member and at a distal part of the tubular member 10, respectively. The first and second cap fastening members are identical to each other, but mirrored in the plane of symmetry.

In the embodiment shown in Fig.2, the first cap fastening member 62 and the second cap fastening member 64 may be configured as circumferentially extending annular protrusions in order to engage a corresponding structure of a cap with which the needle shield remover 10 is going to be assembled. However, the cap fastening members could have many other shapes, as deemed suitable by a skilled person, as long as the connection is made strong enough to withstand the longitudinal force required to remove a needle shield from a needle. Examples of other cap fastening member configurations are snap-fits, ledges, hooks, bumps, grooves, etc.

The plurality of structural features may comprise first shield gripping members 22 and second shield gripping members 24 symmetrically disposed, in relation to the plane of symmetry, at a proximal part of the tubular member and at a distal part of the tubular member 10, respectively. The first and second cap fastening members are identical to each other, but mirrored in the plane of symmetry.

The first shield gripping member 22 and the second shield gripping member 24 may be configured as cut-outs, or tongues, formed out of a circumferential wall of the tubular body 10. They are resilient, radially inwardly extending members, extending towards the plane of symmetry, and configured to engage a distal end, or a circumferential side surface, of a needle shield of a medicament delivery device. The structure of individual gripping members is conventional with regard to the state of the art.

When the medicament delivery device is assembled, the needle shield remover is first attached to a cap by means of the proximal (e.g. first) cap fastening member. In accordance with the present disclosure, and thanks to the symmetrical configuration of the needle shield remover, the tubular body 10 of the needle shield remover may be rotated in either longitudinal direction towards the cap, making the assembly step simple and quick. The cap comprising the needle shield remover is intended to be mounted over the proximal end of a medicament delivery device, to protect a delivery member, e.g. an injection needle.

Secondly, the cap, comprising the needle shield remover 10, is mounted on the medicament delivery device having the injection needle and the needle shield, such that the tubular body 10 of the needle shield remover slides axially along the needle shield until the distal (e.g. second) shield gripping member 24 engages an engagement member on the circumferential side surface of the needle shield, or passes the distal end of the needle shield. The needle shield is somewhat conical, having a distal end which is wider that a proximal end, such that the distal shield gripping member is pushed radially outwards as the tubular body 10 moves distally in relation to the needle shield. When the shield gripping member passes the distal end of the needle shield, the distal shield gripping member relaxes radially inwards and snaps in behind the needle shield remover. Since the distal shield gripping member extends toward the plane of symmetry, it extends proximally, towards a distally-facing end surface of the needle shield. The distally-facing end surface of the needle shield is thereby engaged by the distal shield gripping member.

If a user subsequently removes the cap 50 from the medicament delivery device, the engagement of the proximal cap fastening member to the cap, and the engagement of the distal shield gripping member to the needle shield, will remove the needle shield from the injection needle, and consequently from the medicament delivery device, making the injection needle ready for use.

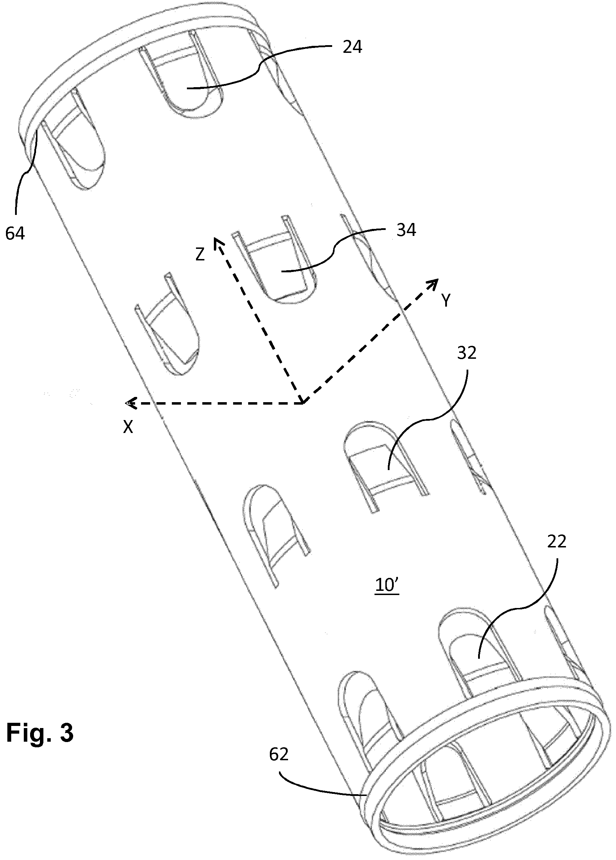

In a second alternative embodiment, shown in Fig. 3, the plurality of structural features further comprises a third shield gripping member 32 and a fourth shield gripping member 34 symmetrically disposed on the tubular body 10', closer to the plane of symmetry than the first shield gripping member 22 and the second shield gripping member 24, respectively. As such, the third shield gripping member 32 and the fourth shield gripping member 34 may be used to either provide an additional grip on a needle shield, i.e. in addition to the first shield gripping member 22 or to the second shield gripping member 24, respectively, or to engage a circumferential side surface or a distal end of a needle shield which is too short to be properly engaged by the more distal shield gripping member.

The assembly steps of this second alternative embodiment are similar to the assembly steps described above.

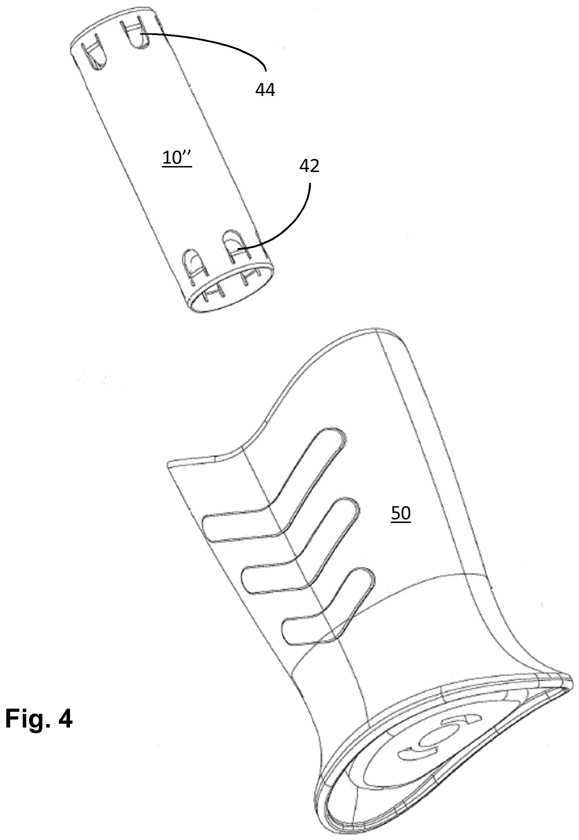

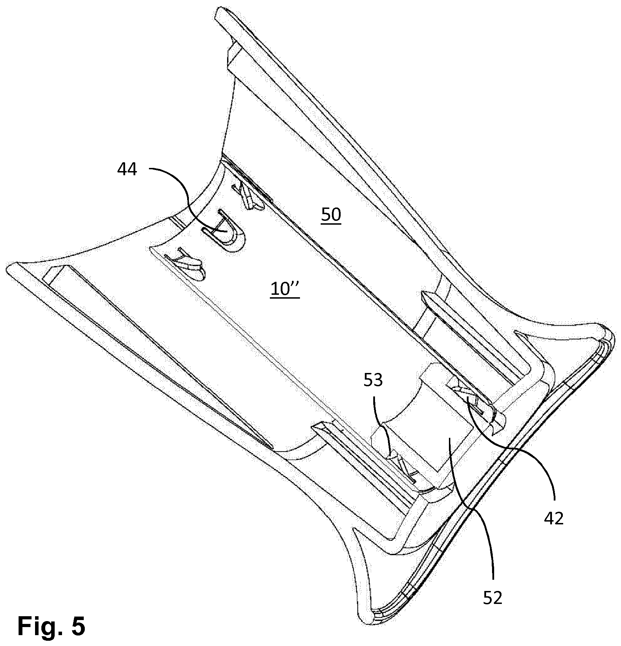

In a third alternative embodiment, shown in Figs 4 and 5, the plurality of structural features comprises a first combined fastening/gripping member 42 and a second combined fastening/gripping member 44 symmetrically disposed, in relation to the plane of symmetry, at a proximal part of the tubular member 10" and at a distal part of the tubular member 10", respectively, such that a combined fastening/gripping member (42, 44) may be used to attach the needle shield remover to a cap of a medicament delivery device.

The first and second combined fastening/gripping members are identical to each other, but mirrored in the plane of symmetry. The first combined fastening/gripping member 42 replaces the first cap fastening member 62 and the first shield gripping member 22 such that the first combined fastening/gripping member 42 may be used either to attach the needle shield remover to an attachment member 52 of a cap 50, or to engage the circumferential side surface or the distal end of a needle shield, depending on the orientation of the tubular body 10" of the needle shield remover. In accordance with the description above, the second combined fastening/gripping member 44 replaces the second cap fastening member 64 and the second shield gripping member 24 such that the second combined fastening/gripping member 44 may be used either to attach the needle shield remover to an attachment member 52 of a cap 50, or to engage the distal end of a needle shield.

Similar to the description above, the combined fastening/gripping members may be configured as cut-outs, or tongues, formed out of a circumferential wall of the tubular body 10". They are resilient, radially inwardly extending members, extending towards the plane of symmetry, and configured to engage a circumferential side surface or a distal end of a needle shield of a medicament delivery device, or an attachment member 52 of a cap 50. The structure of individual combined fastening/gripping members is conventional with regard to the shield gripping members of the state of the art. Hence, structurally, the combined fastening/gripping member is identical to the shield gripping member described above. However, the combined fastening/gripping members may be located closer to the distal and longitudinal ends of the tubular body 10" of the needle shield remover in order to improve the engagement and/or attachment to the attachment member 52 of the cap 50.

The cap 50 comprises an attachment member 52 configured to enable engagement to a proximal combined fastening/gripping member of the needle shield remover, such that the cap 50 and the needle shield remover are rotationally movable, but axially fixed relative to each other.

The attachment member 52 comprises a proximally-facing annular surface 53 with which a combined fastening/gripping member may engage.

The proximally-facing annular surface 53 of the attachment member 52 thereby essentially mimics the end surface of the needle shield in order to enable engagement with a corresponding shield gripping member (i.e. combined fastening/gripping member).

## Claims

1. A needle shield remover for removing a needle shield, wherein the needle shield remover comprises a longitudinally elongated tubular body (10, 10', 10") having a plurality of structural features, **characterized in that** said plurality of structural features are symmetrically disposed on the tubular body (10, 10', 10") in relation to a plane of symmetry which is perpendicular to a longitudinal axis and located at mid-length of the tubular body (10, 10', 10"), wherein the plurality of structural features comprises a first cap fastening member (62) and a second cap fastening member (64) symmetrically disposed, in relation to the plane of symmetry, at a proximal part of the tubular body (10, 10', 10") and at a distal part of the tubular body (10, 10', 10"), and wherein the plurality of structural features further comprises a first shield gripping member (22) and a second shield gripping member (24) symmetrically disposed on the tubular body (10, 10', 10") proximally of the plane of symmetry and distally of the plane of symmetry.

2. The needle shield remover according to claim 1, wherein the plurality of structural features are configured as first combined cap fastening/shield gripping member (42) and a second combined cap fastening/shield gripping member (44) symmetrically disposed, in relation to the plane of symmetry, at a proximal part of the tubular body (10") and at a distal part of the tubular body (10") such that the combined cap fastening/shield gripping members (42, 44) may be used to attach the needle shield remover to a cap (50) of a medicament delivery device.

3. The needle shield remover according to claim 1, wherein the plurality of structural features further comprises a third shield gripping member (32) and a fourth shield gripping member (34) symmetrically disposed on the tubular body (10') closer to the plane of symmetry than the first shield gripping member (22) and the second shield gripping member (24).

4. The needle shield remover according to claim 1 or 3 wherein the first, second, third and fourth shield gripping member (22, 24, 32, 34) is a radially inwardly extending member extending towards the plane of symmetry and configured to engage a circumferential side surface or a distal end of the needle shield of a medicament delivery device.

5. The needle shield remover according to claim 2, wherein the first combined cap fastening/shield gripping member (42) and the second combined cap fastening/shield gripping member (44) are radially inwardly extending members, extending towards the plane of symmetry, and configured to engage an attachment member (52) of the cap (50) for the medicament delivery device or a circumferential side surface or the distal end of the needle shield of the medicament delivery device.

6. A cap (50) for a medicament delivery device, **characterized in that** the cap (50) comprises a needle shield remover according to any of the previous claims.

7. The cap (50) according to claim 6, wherein the cap (50) is configured to be mounted over the proximal end of the medicament delivery device to protect a delivery member, e.g. an injection needle.

8. A method of assembly of the needle shield remover according to claims 1-5 with a cap (50) for a medicament delivery device **characterized by** longitudinally orient the needle shield in either direction along a longitudinal axis of the cap (50) and subsequently attach the needle shield remover to the cap (50).

9. A medicament delivery device comprising a cap (50) **characterized in that** the cap (50) comprises a needle shield remover according to any of claims 1-5.

## Patentansprüche

1. Nadelschutzentferner zum Entfernen eines Nadelschutzes, wobei der Nadelschutzentferner einen in Längsrichtung langgestreckten rohrförmigen Körper (10, 10', 10") umfasst, der eine Vielzahl von Strukturmerkmalen aufweist, **dadurch gekennzeichnet, dass** die Vielzahl von Strukturmerkmalen in Bezug auf eine Symmetrieebene, die senkrecht zu einer Längsachse verläuft und sich auf halber Länge des rohrförmigen Körpers (10, 10', 10") befindet, an dem rohrförmigen Körper (10, 10', 10") symmetrisch angeordnet ist, wobei die Vielzahl von Strukturmerkmalen ein erstes Kappenbefestigungselement (62) und ein zweites Kappenbefestigungselement (64) umfasst, die in Bezug auf die Symmetrieebene an einem proximalen Teil des rohrförmigen Körpers (10, 10', 10") und an einem distalen Teil des rohrförmigen Körpers (10, 10', 10") symmetrisch angeordnet sind, und wobei die Vielzahl von Strukturmerkmalen ferner ein erstes Schutzgreifelement (22) und ein zweites Schutzgreifelement (24) umfasst, die proximal der Symmetrieebene und distal der Symmetrieebene an dem rohrförmigen Körper (10, 10', 10") symmetrisch angeordnet sind.

2. Nadelschutzentferner nach Anspruch 1, wobei die Vielzahl von Strukturmerkmalen als ein erstes kombiniertes Kappenbefestigungs-/Schutzgreifelement (42) und ein zweites kombiniertes Kappenbefestigungs-/Schutzgreifelement (44) ausgebildet ist, die in Bezug auf die Symmetrieebene an einem proximalen Teil des rohrförmigen Körpers (10") und an einem distalen Teil des rohrförmigen Körpers (10") derart symmetrisch angeordnet sind, dass die kombinierten Kappenbefestigungs-/Schutzgreifelemente (42, 44) verwendet werden können, um den Nadelschutzentferner an einer Kappe (50) einer Medikamentenabgabevorrichtung anzubringen.

3. Nadelschutzentferner nach Anspruch 1, wobei die Vielzahl von Strukturmerkmalen ferner ein drittes Schutzgreifelement (32) und ein viertes Schutzgreifelement (34) umfasst, die näher zu der Symmetrieebene als das erste Schutzgreifelement (22) und das zweite Schutzgreifelement (24) an dem rohrförmigen Körper (10') symmetrisch angeordnet sind.

4. Nadelschutzentferner nach Anspruch 1 oder 3, wobei das erste, das zweite, das dritte und das vierte Schutzgreifelement (22, 24, 32, 34) ein sich radial nach innen erstreckendes Element ist, das sich zu der Symmetrieebene hin erstreckt und konfiguriert ist, um eine umlaufende Seitenoberfläche oder ein distales Ende des Nadelschutzes einer Medikamentenabgabevorrichtung in Eingriff zu nehmen.

5. Nadelschutzentferner nach Anspruch 2, wobei das erste kombinierte Kappenbefestigungs-/Schutzgreifelement (42) und das zweite kombinierte Kappenbefestigungs-/Schutzgreifelement (44) sich radial nach innen erstreckende Elemente sind, die sich zu der Symmetrieebene hin erstrecken und konfiguriert sind, um ein Anbringungselement (52) der Kappe (50) für die Medikamentenabgabevorrichtung oder eine umlaufende Seitenoberfläche oder das distale Ende des Nadelschutzes der Medikamentenabgabevorrichtung in Eingriff zu nehmen.

6. Kappe (50) für eine Medikamentenabgabevorrichtung,
**dadurch gekennzeichnet, dass** die Kappe (50) einen Nadelschutzentferner nach einem der vorstehenden Ansprüche umfasst.

7. Kappe (50) nach Anspruch 6, wobei die Kappe (50) konfiguriert ist, um über dem proximalen Ende der Medikamentenabgabevorrichtung montiert zu werden, um ein Abgabeelement, z. B. eine Injektionsnadel, zu sichern.

8. Verfahren für einen Zusammenbau des Nadelschutzentferners nach den Ansprüchen 1 bis 5 mit einer Kappe (50) für eine Medikamentenabgabevorrichtung, **gekennzeichnet durch** ein Längsausrichten des Nadelschutzes in jeder Richtung entlang einer Längsachse der Kappe (50) und ein anschließendes Anbringen des Nadelschutzentferners an der Kappe (50).

9. Medikamentenabgabevorrichtung, umfassend eine Kappe (50),
**dadurch gekennzeichnet, dass** die Kappe (50) einen Nadelschutzentferner nach einem der Ansprüche 1 bis 5 umfasst.

## Revendications

1. Extracteur de protection d'aiguille permettant d'extraire une protection d'aiguille, dans lequel l'extracteur de protection d'aiguille comprend un corps tubulaire (10, 10', 10") allongé longitudinalement ayant une pluralité de caractéristiques structurelles, **caractérisé en ce que** ladite pluralité de caractéristiques structurelles sont disposées symétriquement sur le corps tubulaire (10, 10', 10") par rapport à un plan de symétrie qui est perpendiculaire à un axe longitudinal et situé à mi-longueur du corps tubulaire (10, 10', 10"), dans lequel la pluralité de caractéristiques structurelles comprend un premier élément de fixation de capuchon (62) et un second élément de fixation de capuchon (64) disposés symétriquement, par rapport au plan de symétrie, au niveau d'une partie proximale du corps tubulaire (10, 10', 10") et au niveau d'une partie distale du corps tubulaire (10, 10', 10"), et dans lequel la pluralité de caractéristiques structurelles comprend en outre un premier élément de préhension de protection (22) et un deuxième élément de préhension de protection (24) disposés symétriquement sur le corps tubulaire (10, 10', 10") de manière proximale par rapport au plan de symétrie et distale par rapport au plan de symétrie.

2. Extracteur de protection d'aiguille selon la revendication 1, dans lequel la pluralité de caractéristiques structurelles sont conçues en tant que premier élément combiné de fixation de capuchon/préhension de protection (42) et un second élément combiné de fixation de capuchon/préhension de protection (44) disposés symétriquement, par rapport au plan de symétrie, au niveau d'une partie proximale du corps tubulaire (10") et au niveau d'une partie distale du corps tubulaire (10"), de telle sorte que les éléments combinés de fixation de capuchon/préhension de protection (42, 44) peuvent être utilisés pour lier l'extracteur de protection d'aiguille à un capuchon (50) d'un dispositif d'administration de médicaments.

3. Extracteur de protection d'aiguille selon la revendication 1, dans lequel la pluralité de caractéristiques structurelles comprend en outre un troisième élément de préhension de protection (32) et un quatrième élément de préhension de protection (34) disposés symétriquement sur le corps tubulaire (10') plus près du plan de symétrie que le premier élément de préhension de protection (22) et le deuxième élément de préhension de protection (24).

4. Extracteur de protection d'aiguille selon la revendication 1 ou 3, dans lequel le premier, le deuxième, le troisième et le quatrième élément de préhension de protection (22, 24, 32, 34) est un élément s'étendant radialement vers l'intérieur s'étendant vers le plan de symétrie et conçu pour venir en prise avec une surface latérale circonférentielle ou une extrémité distale de la protection d'aiguille d'un dispositif d'administration de médicaments.

5. Extracteur de protection d'aiguille selon la revendication 2, dans lequel le premier élément combiné de fixation de capuchon/préhension de protection (42) et le second élément combiné de fixation de capuchon/préhension de protection (44) sont des éléments s'étendant radialement vers l'intérieur, s'étendant vers le plan de symétrie, et conçus pour venir en prise avec un élément de liaison (52) du capuchon (50) pour le dispositif d'administration de médicaments ou une surface latérale circonférentielle ou l'extrémité distale de la protection d'aiguille du dispositif d'administration de médicaments.

6. Capuchon (50) pour un dispositif d'administration de médicaments, **caractérisé en ce que** le capuchon (50) comprend un extracteur de protection d'aiguille selon l'une quelconque des revendications précédentes.

7. Capuchon (50) selon la revendication 6, dans lequel le capuchon (50) est conçu pour être monté sur l'extrémité proximale du dispositif d'administration de médicaments pour protéger un élément d'administration, par exemple une aiguille d'injection.

8. Procédé d'assemblage de l'extracteur de protection d'aiguille selon les revendications 1 à 5 avec un capuchon (50) pour un dispositif d'administration de médicaments, **caractérisé par** l'orientation longitudinale de la protection d'aiguille dans l'une ou l'autre direction le long d'un axe longitudinal du capuchon (50) et ensuite la liaison de l'extracteur de protection d'aiguille au capuchon (50).

9. Dispositif d'administration de médicaments comprenant un capuchon (50), **caractérisé en ce que** le capuchon (50) comprend un extracteur de protection d'aiguille selon l'une quelconque des revendications 1 à 5.
